(19) Europäisches Patentamt / European Patent Office / Office européen des brevets

(11) **EP 1 537 664 B1**

(12) **EUROPÄISCHE PATENTSCHRIFT**

(45) Veröffentlichungstag und Bekanntmachung des Hinweises auf die Patenterteilung:
**28.04.2010 Patentblatt 2010/17**

(21) Anmeldenummer: **03750527.8**

(22) Anmeldetag: **11.09.2003**

(51) Int Cl.:
**H03K 17/95** (2006.01)

(86) Internationale Anmeldenummer:
**PCT/EP2003/010126**

(87) Internationale Veröffentlichungsnummer:
**WO 2004/027994 (01.04.2004 Gazette 2004/14)**

(54) **INDUKTIVER SCHALTER**

INDUCTIVE SWITCH

INTERRUPTEUR INDUCTIF

(84) Benannte Vertragsstaaten:
**AT BE BG CH CY CZ DE DK EE ES FI FR GB GR HU IE IT LI LU MC NL PT RO SE SI SK TR**

(30) Priorität: **12.09.2002 DE 10242385**

(43) Veröffentlichungstag der Anmeldung:
**08.06.2005 Patentblatt 2005/23**

(73) Patentinhaber: **ZF Friedrichshafen AG
88038 Friedrichshafen (DE)**

(72) Erfinder:
• **ZAPF, Martin
95473 Creussen (DE)**
• **LUBER, Thomas
92256 Hahnbach (DE)**

(56) Entgegenhaltungen:
EP-A- 0 455 613     EP-A- 0 537 747
DE-A- 2 653 371     DE-A- 3 735 694
DE-A1- 3 743 259    DE-U- 20 011 223
US-A- 4 580 478     US-A- 4 658 153
US-A- 5 532 529     US-B1- 6 179 938

• JAYAWANT B V; WHORLOW R J; WILSON G: "RADIATION-PROOF PROXIMITY SENSOR" MEASUREMENT AND CONTROL, Bd. 32, Nr. 3, 1. April 1999 (1999-04-01), Seiten 80-83, XP000898804 London, GB

EP 1 537 664 B1

**Beschreibung**

Die Erfindung bezieht sich auf eine induktive Sensoreinheit für Schalter, die insbesondere für eine Positionsschaltvorrichtung brauchbar ist, die bei automatischen Fahrzeuggetrieben eingesetzt wird.

Bekannt im Stand der Technik ist ein Weg-Winkel-Sensor, insbesondere zur Bestimmung eines eingelegten Ganges im Kraftfahrzeugbereich, gemäß der Patentschrift DE 198 06 529. Der bekannte Weg-Winkel-Sensor weist vier Messspulen auf, die in einer X-Y-Fläche auf einem Spulenträger in einem Winkel von 90° zueinander angeordnet und an eine Auswerteelektronik angeschlossen sind. Der Sensor weist auch ein sogenanntes Target auf, das im Wesentlichen parallel zu der X-Y-Fläche relativ zu den Messspulen bewegbar ist und dadurch in den Messspulen Spannungen induziert. Aus den induzierten Spannungen kann die Auswerteelektronik den zurückgelegten Weg in Y-Richtung und den Winkel α des Targets in einer Z-X-Fläche ermitteln. Der bekannte Weg-Winkel-Sensor zeichnet sich dadurch aus, dass die jeweils gegenüberliegenden Messspulen in einem Abstand zueinander angeordnet sind und die benachbarten Messspulen sich jeweils zumindest teilweise überlagern.

Die US 5 532 529 offenbart einen induktiven Tastschalter mit einem Verriegelungsmechanismus.

In der älteren Anmeldung gemäß DE 101 25 278 wird ebenfalls die Induktionsspannung als Sensorsignal verwendet. Die bekannten Konstruktionen haben den Nachteil, dass mehrere induktiv wirkende Sensorschleifen auf den Spulenträger aufzubringen sind, wie aus der nachfolgenden Hintergrundinformation ersichtlich.

Aus dem Automobilbereich ist der vielfache Einsatz von mechanischen Schaltern bekannt, unter anderem in Schließsystemen, Bedienelementen der Armaturen, Sitzverstellungen, Spiegelverstellungen usw. Mechanische Schalter haben den Nachteil, daß sie nicht verschleißfrei arbeiten. Ihre Lebensdauer wird einerseits begrenzt durch den Materialabtrag des Kontaktmaterials, durch Materialveränderungen (Oxidation), durch Ablagerungen auf den Schaltkontakten in Folge mechanischer Reibung oder elektrischer Überlastung oder eines Lichtbogens beim Abschaltevorgang.

Eine besondere Form mechanischer Schalter sind mechanische Schleifschalter. Ein verschiebbarer Kontakt läuft über eine Schleifbahn und stellt damit je nach Position einen Kontakt zu wechselnden Anschlüssen her (sogenannte Codierschalter). Im Fahrzeug auftretende Vibrationen führen bei einer derartigen Schaltkulisseneinheit zu einem erhöhten Verschleiß der Schleifkontakte und Schleifbahnen.

In modernen Fahrzeugen werden Verstellmotore heute meist über verschleißfreie Leistungshalbleiter geschaltet, die dann allerdings wieder durch nicht verschleißfreie Schalter angesteuert werden. Um das System vollständig verschleißfrei zu gestalten, ist die Entwicklung von neuartigen Schaltern notwendig, die ohne mechanische Schaltkontakte (also mit Sensoren) arbeiten.

Bekannt im Stand der Technik sind Hall-Sensoren, die auf die Annäherung von Permanentmagneten reagieren und damit eine Schaltfunktion auslösen. Weiterhin bekannt ist die Verwendung von GMR-Sensoren, die auf dem Effekt einer Widerstandsänderung beruhen, die durch ein äußeres Magnetfeld hervorgerufen wird. Das äußere Magnetfeld kann einem Permanentmagneten oder einem magnetisierbaren Kunststoff entstammen und entsprechende Schaltfunktionen veranlassen.

Weiterhin ist der Einsatz von Lichtschranken und Reflexlichtschranken bekannt, die den Nachteil haben, daß sie störlichtempfindlich sind und daß die optischen Bauteile altern und leicht verschmutzen können. Der Einsatz solcher Sensoren hat ferner den Nachteil, dass sie im Vergleich zu mechanischen Schaltern und zu induktiven Schaltern teuer sind.

In Schaltelementen wird als Träger für Beleuchtungen, Anzeigen oder mechanische Schalter oft eine kostengünstige Leiterplatte verwendet. Das Vorhandensein einer solchen Leiterplatte begünstigt den Einsatz der vorliegenden Erfindung. Als kostengünstige Möglichkeit ist das in der älteren Anmeldung gemäß DE 101 25 278 verwendete Wirkungsprinzip der induktiven Kopplung zweier auf der Leiterplatte aufgebrachter Sensorspulen und deren Bedämpfung durch einen leitfähigen Betätiger vorangemeldet. Dabei korreliert die Bedämpfungsstärke mit der Position des Betätigers relativ zu den Sensoren. Nachteilig an dieser Technologie kann sich auswirken, dass die Sensoren in der praktischen Ausführung eine Mindestgröße von ca. 10 mm x 10 mm auf der Leiterplatte haben müssen, damit eine annehmbare Kopplung erreicht werden kann und damit die Aufbereitungselektronik einfach und kosteneffektiv gestaltet werden kann. Bei den derzeit kostengünstig produzierbaren Leiterplatten wird eine örtliche Auflösung von 0,12 mm erreicht, d. h. die Leiterbreite der Sensorwicklungen kann maximal 0,12 mm betragen ebenso wie die Isolierbreite zwischen den Wicklungen. Dadurch bedingt kann die Sendespule wie auch die Empfangsspule der Sensoren nur ca. 5 Windungen aufweisen.

Die Aufgabe der Erfindung besteht darin, die Induktivität einer Sensorspule durch einen über die Spule gebrachten Betätiger zu beeinflussen und diese Induktivität in einfacher Weise auszuwerten. Die Induktivität einer Spule ändert sich erheblich durch ein leitfähiges Betätigungselement, das gemäß dem unabhängigen Anspruch 1 eine veränderliche Überdeckung der Sensorspule aufweist. Die Aufgabe wird durch eine induktive Sensoreinheit mit den Merkmalen gemäß Anspruch 1 gelöst. Zweckmäßige Weiterbildungen sind in den abhängigen Ansprüchen definiert.

Eine ungedämpfte Sensorspule mit dem Außenmaß 10 mm x 10 mm, die auf der Leiterplatte wie eine rechteckige Spirale von innen nach außen gewickelt ist, weist bei der auf der Leiterplatte erzielbaren Auflösung 10 Windungen und eine Induktivität von ca. 1 μH auf.

Zwar ist es aus der Druckschrift GB 1 415 644 bekannt, die Impedanz einer Spiralstruktur als Sensor zu verwenden, jedoch ist die bekannte Spiralstruktur bifilar gewickelt, um die ohmsche Komponente der Spiralimpedanz auszunutzen und um die induktive Komponente der Spiralimpedanz auszuschalten. Im Unterschied dazu ist die Sensorspule gemäß der Erfindung monofilar gewickelt, wie sich aus der nachfolgenden detaillierten Beschreibung anhand der Figuren ergibt.

Figur 1 zeigt die planare Ausführung einer Sensorschleife auf einer Leiterplatte zusammen mit dem elektrotechnisch äquivalenten Symbol.

Figur 2 zeigt ein funktionelles Blockschaltbild eines erfindungsgemäßen Sensors mit einem LC-Schwingkreis als Auswerteschaltung.

Figur 3 zeigt ein funktionelles Blockschaltbild mit einem LC-Schwingkreis und mit zwei Sensoren zur Detektion eines Verschiebewegs y.

Figur 4 zeigt eine typische Kennlinie für die Schwingfrequenz mit einem ersten Sensor L1 gemäß Figur 3 und einem zweiten Sensor L2 gemäß Figur 3 als Funktion des Verschiebeweges y.

Figur 5 zeigt eine Schaltkulisse für ein Kraftfahrzeug mit einem Automatikwählhebel, der an eine Sensoreinheit gemäß Figur 3 angeschlossen ist.

Figur 6 zeigt das Schema einer Leiterplatte mit mehreren Sensoreinheiten für die Schaltkulisseneinheit gemäß Figur 5.

Figur 7 zeigt das Blockschaltbild einer elektronischen Einheit, wenn mehrere induktive Sensoren kombiniert werden.

Figur 8 zeigt die Schwingkreisfrequenz der Sensorsignale der verschiedenen Schalteinheiten in Fig. 7 bei Schaltvorgängen des Automatikwählhebels von der Position 1 bis zur Position 4.

Figur 9 zeigt ein ähnliches Schema einer Leiterplatte wie in Figur 6, jedoch mit einer redundanten Schalteinheit.

Figur 10 zeigt das Blockschaltbild einer alternativen Auswerteeinheit.

Figur 11 zeigt die gemäß Fig. 10 ausgewertete Induktivitätsänderung.

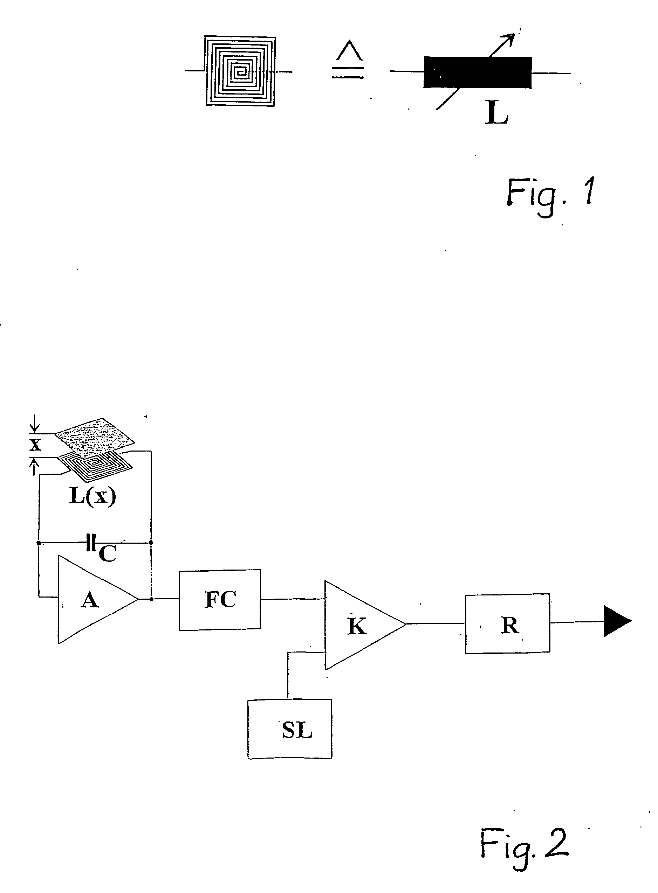

Gemäß Figur 1 ist eine Sensorspule planar auf einer Leiterplatte aufgebracht. Der Anschluß im Mittelpunkt der Spirale ist beispielsweise auf der Rückseite der Leiterplatte herausgeführt. Deckt man den Sensor gemäß Figur 1 mit einem leitfähigen Betätiger im Abstand x von beispielsweise x = 0,05 mm ab, so verringert sich die Induktivität von beispielsweise ca. 1 μH auf beispielsweise ca. 0,2 μH.

Die Verringerung der Induktion durch den Betätiger B ist vom Abstand x des Betätigers B zur Sensorschleife abhängig; sie ist aber auch vom Überdeckungsgrad der Sensorschleife durch das Betätigerelement abhängig. Überdeckt der Betätiger die gesamte Fläche der Schleife in einem konstanten Abstand x, so wird die Amplitude der Sensorspannung bei dem Überdeckungsgrad von 100 % minimal, wobei die Größe der minimalen Sensorspannung von dem Abstand x abhängt.

Damit sind für den Schalter zwei Schaltmechanismen möglich:

- Der Überdeckungsgrad G wird auf einer definierten Größe gehalten, und der Abstand x zwischen dem Betätigerelement B und der Sensorschleife wird variiert (wie es z.B. in Figur 2 dargestellt ist), oder

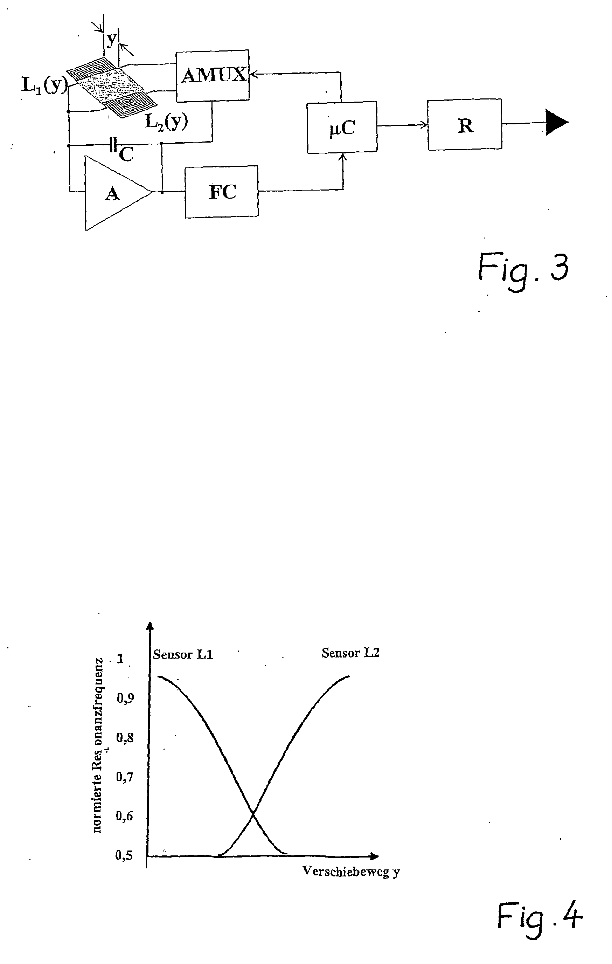

- der Abstand x wird konstant gehalten, und der Überdeckungsgrad G wird verändert (wie es z.B. in Figur 3 dargestellt ist).

- Auch eine Kombination der beiden Schaltmechanismen ist möglich.

Als kostengünstige Auswerteelektronik hinlänglich bekannt ist ein LC-Schwingkreis, bestehend aus einer Sensorinduktivität L, einer Festkapazität C und einem invertierenden Verstärker A, in dessen Rückkopplungszweig der LC-Schwingkreis eingebaut ist. Eine derartige Schaltung ist in Figur 2 als Blockschaltbild dargestellt. Die Frequenz des Schwingkreises wird durch die Resonanzfrequenz des LC-Gliedes bestimmt nach der Formel:

$$ f_r = \frac{1}{2\pi} \; x \; \frac{1}{\sqrt{LC}} \; . $$

Ein nachgeschalteter Frequenzzähler FC bestimmt gemäß Figur 3 die Schwingungen pro Zeiteinheit und gibt sie als Signalwert aus. Für eine einfache Schaltfunktion genügt es, mittels eines Komparators den aktuellen Frequenzwert mit einem Schwellwert zu vergleichen und damit die Schaltfunktion auszulösen. In einem gewöhnlichen Fall wird das Schaltsignal auf "1" gesetzt, wenn die Frequenz höher ist als eine eingestellte Grenzfrequenz, was einer geringeren Induktivität durch eine höhere Bedämpfung entspricht. Bei einer geringeren Frequenz gibt der Komparator eine "0" aus. Über einen nachgeschalteten Hoch-Niedrig-Schalter oder ein Relais R können dann hohe Leistungen geschaltet werden. Die Funktionen Frequenzzähler und Komparator können aber auch als sogenannte Firmware in einem Mikrocontroller realisiert werden.

Damit kann auf einfache Weise ein verschleißfreier Tastschalter in einer Bedieneinheit des Automobils realisiert werden. Das Bedämpfungselement wird gemäß Figur 2 durch Druck auf eine Taste an den Sensor angenähert und dort mittels eines Verriegelungsmechanismus gehalten. Erst bei erneutem Druck auf den Tastenknopf wird die Verriegelung aufgehoben, und der Betätiger wird in seine Ruheposition in einem größeren Abstand zum Sensor gebracht (Kugelschreiberverriegelungsprinzip). So lassen sich auf einfache und kostengünstige Weise Schalttaster wie etwa der Schalter für Warnblinker, Nebelleuchten, Heckscheibenheizung usw. realisieren.

In Applikationen, in denen sehr genaue Schaltpunkte erforderlich sind, sind oft die Temperatureinflüsse auf Verstärker, Kapazitäten, Komparatoren usw. problematisch. In temperaturstabilen Applikationen kann man diese Einflüsse umgehen, indem man zwei Sensoren nebeneinander auf einer Leiterplatte aufbringt und beide wechselseitig in den Schwingkreis einschaltet (vgl. Figur 3). Das Zuschalten der Induktivität L1 oder L2 geschieht durch einen Schalttransistor oder Feldeffekttransistor oder MOSFET oder einen Analogmultiplexer AMUX. Wendet man eine relative Auswertung an, indem man als Schaltkriterium das Frequenzverhältnis der ersten Sensorfrequenz zur zweiten Sensorfrequenz verwendet, so fallen die Störeinflüsse heraus. Die Schaltung ist sehr temperaturstabil.

Diese Schaltungsart erweist sich auch bei Applikationen als vorteilhaft, bei denen die Position y des Betätigers relativ zu den Sensorpositionen detektiert wird, während der Abstand x des Betätigers zum Sensor mehr oder weniger konstant gehalten wird (wie z.B. bei Weg-Winkel-Sensoren). Auch hier findet gemäß Figur 3 eine relative Auswertung statt, die am besten, jedoch nicht ausschließlich, durch einen Mikrocontroller $\mu$C erfolgen kann.

Figur 4 zeigt zwei typische Kennlinien der normierten Resonanzfrequenz als Funktion des Verschiebewegs y. In dem Verschiebebereich zwischen den Maxima der Kennlinien L1 und L2 kann der Mikrocontroller $\mu$C eine exakte Positionserkennung vornehmen. In weiteren praktische Anwendungsfällen können noch mehr Sensoren zur Erkennung der Betätigerposition verwendet werden.

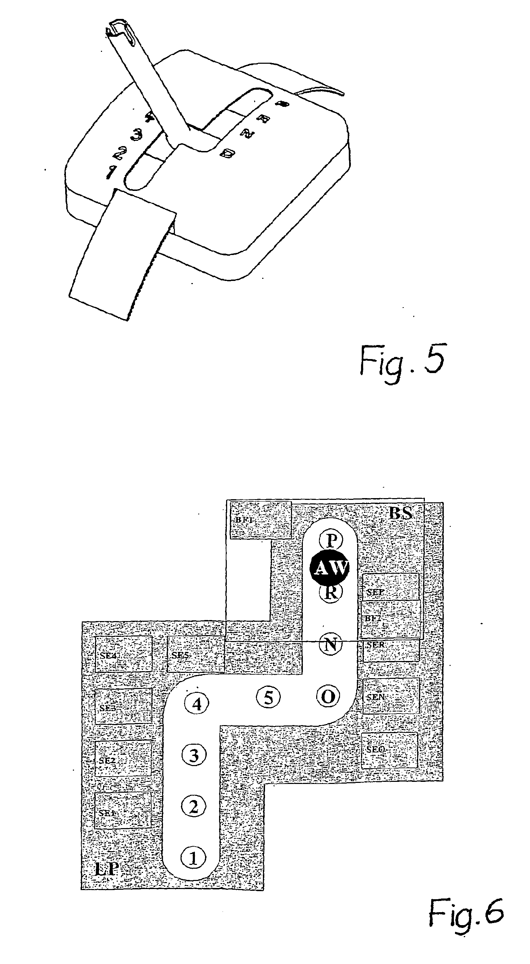

Sind in einem Anwendungsfall, wie er in Figur 5 als Schaltkulisse für ein Kraftfahrzeug dargestellt ist, mehrere Positionen zu detektieren, so ist es zweckmäßig, mehrere induktive Sensoren als Funktionseinheit zu kombinieren. Am Beispiel der Umsetzung der Positionserkennung eines Automatikwählhebels sieht das wie folgt aus:

Unter der Blende wird eine Leiterplatte wie in Figur 5 positioniert, auf deren Oberseite z.B. die Hinterleuchtung der Blendenanzeigen 1, 2, ... P montiert werden kann. Mit dem Automatikwählhebel AW (siehe Figur 6), der durch einen Ausbruch in der Leiterplatte taucht, ist ein Betätigerschlitten BS verbunden, der auf der Unterseite der Leiterplatte LP plan aufliegt und auf dem ein Betätiger oder mehrere Betätiger (in Figur 6 z.B. die Betätigerflächen BF1 und BF2) angebracht sind. Die Betätigerflächen werden in einem definierten Abstand über die verschiedenen Sensoreinheiten SE geschoben.

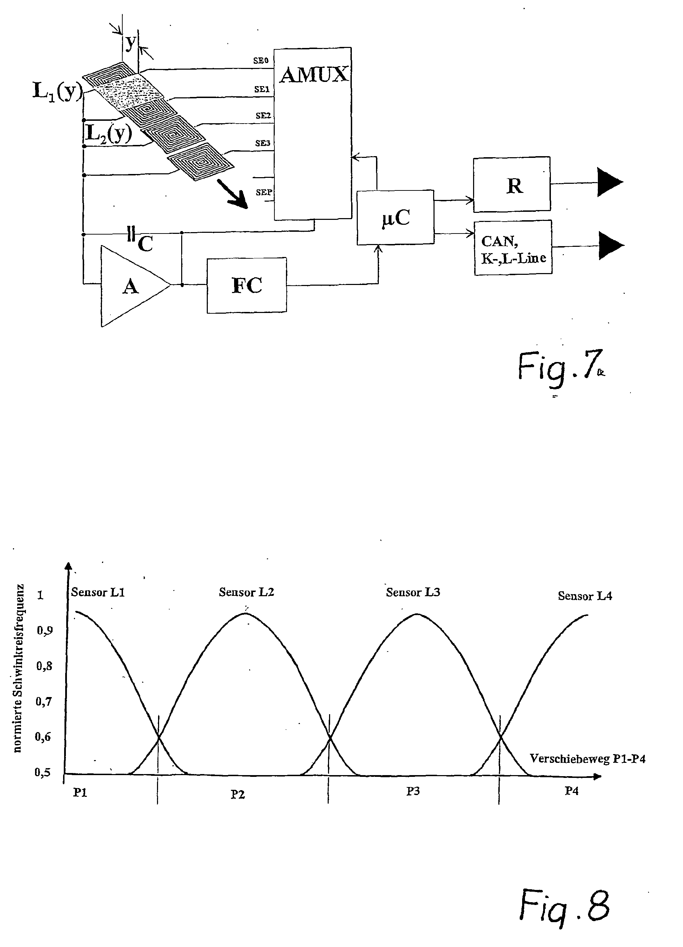

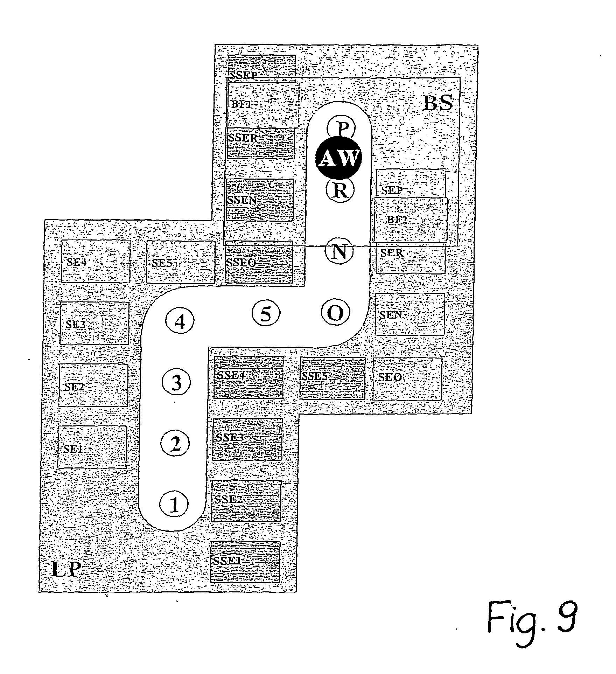

Mit dem Begriff der Verschiebung ist eine Bewegung des Betätigerschlittens gemeint, die geradlinig sein oder die Richtung wechseln kann. Die zu überstreichenden Sensorspulen können auf einer geraden Linie aufgereiht sein, wie es prinzipiell in Figur 7 dargestellt ist. Die Sensorspulen können jedoch auch gemäß einer komplexeren Topologie nebeneinander liegen, wie sie beispielsweise für Schaltkulissen gemäß den Figuren 6 und 9 angewendet wird.

Ferner kann die Topologie der benachbarten Sensorspulen (Gerade, Polygonzug, sonstige Bahn) auf einer ebenen oder auf einer gekrümmten Leiterplatte liegen. In Figur 7 ist eine ebene Anordnung von Sensorspulen dargestellt, während in Figur 5 und 6 ein Beispiel für eine gekrümmte Leiterplatte dargestellt ist, die unter der gebogenen Schaltkulisse angeordnet ist. In beiden Fällen wird der Betätigerschlitten in einem Abstand, der im wesentlichen konstant ist, oberhalb der Anordnung von Sensorspulen verschoben.

Eine weitere Variante der beanspruchten Verschiebung ergibt sich, wenn man die gekrümmte Leiterplatte, die gemäß Figur 5 der gebogenen Kulisse folgt, durch eine ebene Leiterplatte ersetzt, die in einer senkrecht zur Fläche der Kulisse laufenden Ebene angeordnet ist. In diesem Fall wird auch der Betätigerschlitten senkrecht gestellt und nacheinander über Sensorschleifen geschoben, die z.B. kreisbogenförmig auf der senkrechten, ebenen Leiterplatte aufgebracht sind.

Bei der Kombination mehrerer induktiver Schalter stellt sich das Blockschaltbild wie in Figur 7 dar. Die zugehörigen Amplituden der Sensorsignale bei Schaltvorgängen des Automatikwählhebels sind in Figur 8 für die Positionen 1, 2, 3 und 4 zu erkennen, wobei die normierte Schwingkreisfrequenz über dem Verschiebeweg P1 - P4 für die Sensoren L1 - L4 aufgetragen ist. Die jeweiligen Umschaltschwellen P1 - P2, P2 -P3 und P3 - P4 sind eingetragen.

Auch eine sehr redundante und damit sichere Positionserkennung ist ohne großen Zusatzaufwand zu realisieren, wie z.B. in Figur 9 dargestellt. Es wird vorgeschlagen, statt einer Sensoreinheit pro Position zwei Sensoreinheiten pro Position aufzubauen und die Signale zu vergleichen. Bei widersprüchlichen Ergebnissen wird die Auswerteeinheit die Schaltfunktion so ausführen, daß das gesamte System in einem sicheren Zustand gebracht wird. Die Leiterplatte kann dazu beispielsweise mit Sicherheitssensoreinheiten SSE gemäß Figur 9 erweitert werden.

Die Auswerteeinheit für das Sensormodul wird in der Regel ein Mikrocontroller sein, der über eine Schnittstelle (CAN, LIN, etc.) die Schaltinformationen an die Steuerelektronik bzw. Leistungselektronik weitergibt.

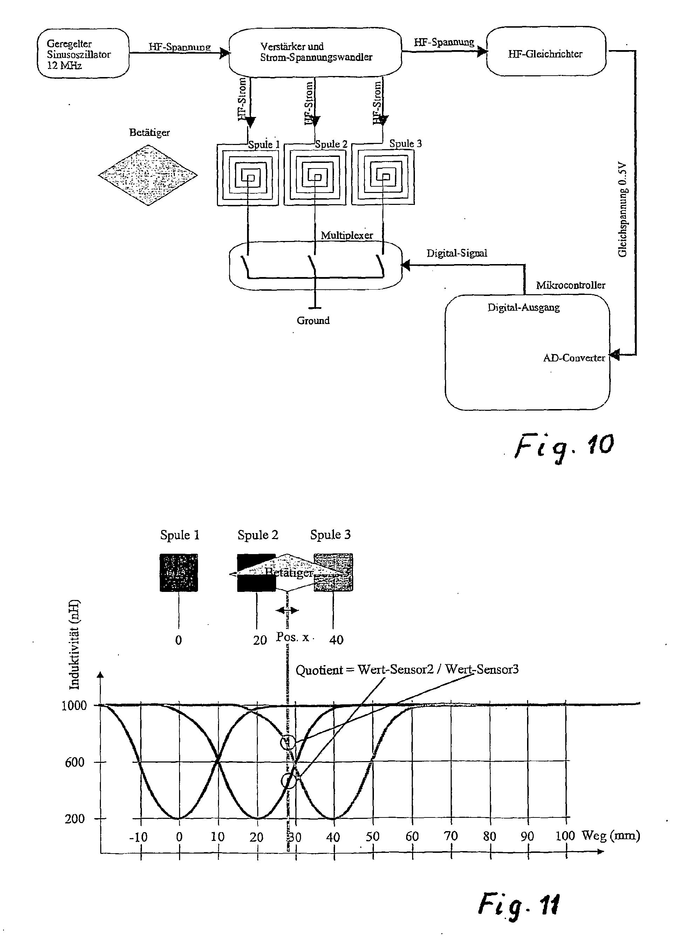

Die Signalauswertung erfolgt bei mehreren Sensorspulen über einen Multiplexer, der nur jeweils eine der Sensorspulen der Auswerteschaltung zuschaltet. Hierfür zeigen die Fig. 10 und 11 ein weiteres Ausführungsbeispiel der Signalauswertung, das alternativ zu dem in den Fig. 7 und 8 dargestellten Ausführungsbeispiel eingesetzt werden kann.

Gemäß Fig. 10 und 11 wird die Grenze zwischen den Positionen zweier benachbarter Sensorspulen durch einen direkten Vergleich der jeweiligen induktiven Reaktanz festgestellt. Nach dem Blockschaltbild in Fig. 10 erzeugt ein Sinusoszillator eine Wechselspannung von 12 MHz; diese wird verstärkt und als Spannung in je eine der drei Sensorspulen, die im Zeitmultiplex eingeschaltet werden, eingespeist. Bewegt man den Betätiger, der aus leitfähigem Material besteht, über die Spulen, verringert sich durch Wirbelstromverluste die Induktivität der Spulen. Dadurch verringert sich auch die Reaktanz, die folgendermaßen berechnet wird: XL = 2 * PI * f * L (f = 12 MHz). Hat zum Beispiel eine Spule eine Induktivität von 1000nH ohne Betätiger und 200nH mit Betätiger, so folgt aus obiger Gleichung ein XL von 75 Ohm, bzw. 15 Ohm. Bei konstanter Wechselspannung fließt ein Strom, der von einer Überdeckung des Betätigers abhängt. Dieser Strom wird in eine proportionale HF-Spannung umgewandelt und gleichgerichtet dem Mikrocontroller zugeführt.

Das Auswertekonzept des Mikrocontrollers ist in Fig. 11 dargestellt. Der Mikrocontroller misst zyklisch in festen Zeitschlitzen von z. B. 2ms die Spannung jedes Sensors. Bewegt man den Betätiger in festen Weg-Schritten über die Sensoren und zeichnet die Spannungen auf, entstehen die Kurven wie in Fig. 11 gezeigt. Mit zusätzlichen Sensoren kann der Wegbereich vergrößert werden. Im statischen Zustand des Betätigers werden immer nur 2 Werte ausgewertet, d.h. für den Mikrocontroller sind zu jedem Zeitpunkt nur 2 benachbarte Messwerte vorhanden. In der Auswertefirmware im Mikrocontroller wird der Quotient aus zwei benachbarten Sensorspannungen berechnet und mit einem festen Wert verglichen. Je nachdem, ob der Quotient größer oder kleiner als der feste Wert ist, wird die eine oder die andere Schaltposition ermittelt.

**Patentansprüche**

1. Induktive Positionsschaltvorrichtung mit einem Wählhebel (AW), einem Betätigerschlitten (BS), einer Sensoreinheit (L1, L2, y) und einer Auswerteschaltung (C, A, FC, u. C, AMUX, R), wobei die Sensoreinheit versehen ist mit mindestens zwei Sensorspulen (L1, L2 ; SE1, SE2, SE3, SE4, SE5, SEO, SEN, SER, SEP), die nebeneinander auf einer Leiterplatte (LP) aufgebracht sind, und mit mindestens einem leitfähigen Betätigungselement (BF1, BF2) an dem Betätigerschlitten (BS), dessen Überdeckung je zweier der Sensorspulen (L1, L2, usw. ) zur Änderung ihrer Induktivitäten durch Verschiebung des Betätigerschlittens (BS) veränderbar ist, wobei die Induktivitätsänderungen der nebeneinander aufgebrachten Sensorspulen (L1, L2 ; SE1, SE2, SE3, SE4, SE5, SEO, SEN, SER, SEP) in der Auswerteschaltung (C, A, FC, IC, AMUX, R) Schaltfunktionen auslösen, und wobei zur Bildung eines temperaturstabilen und exakten Schaltkriteriums wechselseitig nur jeweils eine der Sensorspulen (L1 oder L2 usw. ) der Auswerteschaltung (C, A, FC, u. C, AMUX, R) zugeschaltet ist.

2. Induktiver Schalter nach Anspruch 1, **dadurch gekennzeichnet, dass** die Signalauswertung der Sensorspulen über einen Multiplexer erfolgt.

3. Induktiver Schalter nach einem der Ansprüche 1 bis 2, **gekennzeichnet durch** einen Einbau der induktiven Sensorspule in einen LC-Schwingkreis.

4. Induktiver Schalter nach Anspruch 3, **gekennzeichnet durch** eine Auswertung der Resonanzfrequenz des LC-Schwingkreises, in welche die veränderliche Induktivität eingeht.

5. Induktiver Schalter nach einem der Ansprüche 1 bis 2, **gekennzeichnet durch** die Einprägung einer Wechselspannung konstanter Amplitude und konstanter Frequenz (f) in die Sensorspule mit anschließender Auswertung der Stromamplitude der veränderlichen Induktivität.

**Claims**

1. Inductive position-switching device having a selector lever (AW), an activator carriage (BS), a sensor unit (L1, L2, y) and an evaluation circuit (C, A, FC, μC, AMUX, R), wherein the sensor unit is provided with at least two sensor coils (L1, L2; SE1, SE2, SE3, SE4, SE5, SEO, SEN, SER, SEP) which are mounted one next to the other on a printed circuit board (LP), and having at least one conductive activation element (BF1, BF2) on the activator carriage (BS), the coverage of which by in each case, two of the sensor coils (L1, L2, etc.) can be changed in order to change their inductances by shifting the activator carriage (BS), wherein the changes in inductance of the sensor coils (L1, L2; SE1, SE2, SE3, SE4, S45, SEO, SEN, SER, SEP) which are mounted one next to the other in the evaluation circuit (C, A, FC, IC, AMUX, R) trigger switching functions, and wherein, in order to form a precise switching criterion which is stable with respect to temperature, in each case only one of the sensor coils (L1, or L2 etc.) of the evaluation circuit (C, A, FC, μC, AMUX, R) is alter-

nately activated.

2. Inductive switch according to Claim 1, **characterized in that** the signal evaluation of the sensor coils is carried out by means of a multiplexer.

3. Inductive switch according to one of Claims 1 to 2, **characterized by** installation of the inductive sensor coil in an LC oscillatory circuit.

4. Inductive switch according to Claim 3, **characterized by** evaluation of the resonant frequency of the LC oscillatory circuit, which evaluation includes the variable inductance.

5. Inductive switch according to one of Claims 1 to 2, **characterized by** the impression of an alternating voltage of constant amplitude and constant frequency (f) onto the sensor coil with subsequent evaluation of the current amplitude of the variable inductance.

**Revendications**

1. Dispositif à interrupteur de position inductif avec un levier de sélection (AW), un coulisseau d'actionneur (BS), une unité de détection (L1, L2, y) et un circuit d'analyse (C, A, FC, μC, AMUX, R), l'unité de détection étant pourvue d'au moins deux bobines de capteur (L1, L2 ; SE1, SE2, SE3, SE4, SE5, SEO, SEN, SER, SEP), disposées côte à côte sur une plaque conductrice (LP) et d'au moins un élément d'actionnement (BF1, BF2) conducteur au niveau du coulisseau actionneur (BS), le chevauchement de deux des bobines de capteur (L1, L2, etc.) pouvant respectivement être modifié pour modifier leur inductances par déplacement du coulisseau d'actionneur (BS), les variations d'inductance des bobines de capteur (L1, L2 ; SE1, SE2, SE3, SE4, SE5, SEO, SEN, SER, SEP) placées côte à côte déclenchant des fonctions de commutation dans le circuit d'analyse (C, A, FC, IC, AMUX, R) et seule une des bobines de capteur (L1 ou L2 etc.) du circuit d'analyse (C, A, FC, μC, AMUX, R) étant respectivement activée en alternance pour former un critère de commutation exact et stable en matière température.

2. Interrupteur inductif selon la revendication 1, **caractérisé en ce que** l'analyse de signal des bobines de capteur se produit par l'intermédiaire d'un multiplexeur.

3. Interrupteur inductif selon l'une quelconque des revendications 1 à 2, **caractérisé par** une intégration de la bobine de capteur inductive dans un circuit oscillant LC.

4. Interrupteur inductif selon la revendication 3, **carac-**

**térisé par** une analyse de la fréquence de résonance du circuit oscillant LC par lequel passe l'inductance variable.

5. Interrupteur inductif selon l'une quelconque des revendications 1 à 2, **caractérisé par** l'application d'une tension alternative d'amplitude constante et de fréquence constante (f) dans la bobine de capteur avec analyse consécutive de l'amplitude de courant de l'inductance variable.

Fig. 1

L(x)

$\mathbf{x}$

C

A

FC

K

R

SL

Fig. 2

7

Fig. 3

Fig. 4

Fig. 5

Fig. 6

Fig.7a

Fig.8

Fig. 9

*Fig. 10*

*Fig. 11*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19806529
- US 5532529 A
- DE 10125278
- GB 1415644 A